# EUROPÄISCHE PATENTSCHRIFT

(11) **EP 1 924 355 B1**
(45) Veröffentlichungstag und Bekanntmachung des Hinweises auf die Patenterteilung: **27.03.2019**
(21) Anmeldenummer: 06778285.4
(22) Anmeldetag: 21.08.2006
(51) Int. Cl.: B01J 21/18, B01J 23/89, B01J 37/16, B01J 37/02, C07C 209/00

(54) **VERFAHREN ZUR HERSTELLUNG VON AMINEN MIT EINEM KATALYSATOR ENTHALTEND PLATIN, NICKEL UND EINEM ZUSÄTZLICHEN METALL**
METHOD FOR PRODUCING AMINES WITH A CATALYST CONTAINING PLATINUM, NICKEL AND AN ADDITIONAL METAL
PROCEDE DE PRODUCTION D'AMINES AVEC UN CATALYSEUR CONTENANT DU PLATINE, DU NICKEL ET UN METAL SUPPLEMENTAIRE

(30) Priorität: 31.08.2005 DE 102005041532
(43) Veröffentlichungstag der Anmeldung: 28.05.2008
(73) Patentinhaber: BASF SE, 67056 Ludwigshafen am Rhein (DE)
(72) Erfinder: KUBANEK, Petr, 68163 Mannheim (DE); SCHWAB, Ekkehard, 67434 Neustadt (DE); VAN LAAR, Frederik, Dubai (AE); MACKENROTH, Wolfgang, 67089 Bad Dürkheim (DE)
(86) Internationale Anmeldenummer: PCT/EP2006/065478
(87) Internationale Veröffentlichungsnummer: WO 2007/025884

(56) Entgegenhaltungen:
- EP-A- 1 358 935
- US-A- 4 298 462
- US-A- 4 374 046
- US-A1- 2005 227 128

## Beschreibung

Die Erfindung betrifft ein Verfahren zur Herstellung von Aminen durch katalytische Hydrierung der entsprechenden Nitroverbindungen.

Die Herstellung von Aminen, insbesondere von aromatischen Mono-, Di- und/oder Polyaminen durch katalytische Hydrierung der entsprechenden Mono-, Di- und/oder Polynitroverbindungen ist seit langem bekannt und vielfach in der Literatur beschrieben. Ein in der Technik häufig eingesetztes aromatisches Amin ist das Toluylendiamin (TDA), das zu Toluylendiisocyanat weiterverarbeitet werden kann und durch Hydrierung Dinitrotoluol (DNT) hergestellt wird. Ein Problem bei der Hydrierung von DNT ist die verstärkte Bildung von Nebenprodukten, neben Leichtsiedern, zumeist desaminierte und kernhydrierte Produkten, kommen häufig höhermolekulare oder teerartige Produkte vor, die neben der Verringerung der Ausbeute des Verfahrens auch zu einer vorzeitigen Desaktivierung des Katalysators führen können.

Als Hydrierkatalysatoren werden, wie beispielsweise in EP-A-0 124 010 beschrieben, häufig Metalle der VIII. Nebengruppe des Periodensystems, insbesondere Raney-Eisen, Raney-Kobalt und Raney-Nickel, eingesetzt.

Häufig werden für die Hydrierung von Nitroaromaten auch Katalysatoren eingesetzt, die Edelmetalle, insbesondere Palladium, oder auch Platin, enthalten. Bekannt sind hierbei auch Katalysatoren, die Platin und Nickel enthalten.

So beschreibt US 3,127,356 ein Verfahren zur Herstellung von Hydrierkatalysatoren, die für die Hydrierung von DNT zu TDA eingesetzt werden können. Die Katalysatoren enthalten einen Träger, eine oleophile Kohlenstoffkomponente, wie zum Beispiel ein Ruß, auf der die Metalle aufgebracht sind. Dabei liegt das Nickel im Katalysator als Oxid oder Hydroxid vor,

US 5,214,212 beschreibt ein Verfahren zur Kernhydrierung von aromatischen Aminen. Als Katalysator wird ein Edelmetallkatalysator eingesetzt, der zusätzlich mit weiteren Metallen, unter anderem Nickel, dotiert sein kann. Als Edelmetall kann Platin im Gemisch mit anderen Edelmetallen eingesetzt werden. Dabei liegen die Edelmetalle im Katalysator als Metalle und die dotierten Metalle in Form von Salzen vor.

In DE 39 28 329 wird ein Verfahren zur Herstellung von chlorsubstituierten aromatischen Aminen aus den entsprechenden Nitroverbindungen beschrieben. Der bei diesem Verfahren eingesetzte Katalysator besteht aus Aktivkohle als Träger, auf die Platin und ein weiteres Metall, insbesondere Nickel, aufgebracht sind.

In EP 595 124 wird ein Verfahren zur Herstellung von chlorsubstituierten aromatischen Aminen aus den entsprechenden Nitroverbindungen beschrieben. Der verwendete Katalysator enthält Platin und Nickel auf Aktivkohle. Dabei wird zunächst Platin auf der Aktivkohle aufgebracht und reduziert und danach Nickel in Form eines Salzes auf den Träger aufgebracht. Das Nickel liegt bei diesem Katalysator als Hydroxid vor.

In EP 768 917 wird ein Katalysator zur Herstellung von Carbonsäure-Salzen beschrieben. Dieser besteht aus einem Ankermetall, beispielsweise Platin, das teilweise in einem alkaliresistenten Träger eingebettet ist und mindestens teilweise mit einem katalytisch wirkenden nichtedlen Metall, beispielsweise Nickel, durch stromloses Überziehen beschichtet wird. Bei diesem Katalysator liegen die beiden Metalle als getrennte Phasen auf dem Träger vor.

In US 2004 / 0199017 A1 wird ein Monolith Katalysator zur Hydrierung von Dinitrotoluol zur Toluyldiamin beschrieben. Dieser besteht aus einem monolithischen Träger mit Aluminiumoxid Dünnschicht; als aktive Komponente werden Palladium, Nickel und ein Promotor wie zum Beispiel Zink, Kadmium, Kupfer oder Silber beschreiben.

In US 4,185,036 wird ein Verfahren zur Hydrierung von Mischungen von Nitroaromaten beschrieben. Die verwendeten Katalysatoren enthalten Platin und gegebenenfalls ein weiteres Metall, beispielsweise Nickel, auf Aktivkohle. Das weitere Metall liegt dabei in Form des Oxids oder Hydroxids auf dem Träger vor.

DE 199 11 865 und DE 196 36 214 beschreiben Verfahren zur Hydrierung von Dinitrotoluol. Die eingesetzten Katalysatoren enthalten Iridium sowie mindestens ein Dotierungselement, beispielsweise Nickel oder Platin.

WO 03/39743 beschreibt ein Verfahren zur Herstellung von TDA unter Verwendung eines Hydrierkatalysators, bestehend aus Platin, einem weiteren Edelmetall und einem unedlen Metall.

In WO 05/037768 werden Katalysatoren und Verfahren zur Hydrierung von Dinitrotoluol zur Toluyldiamin beschrieben. Die Katalysatoren bestehen aus Platin und Nickel, wobei die beide Metalle auf dem Träger in Form einer Legierung vorliegen.

Ständige Aufgabe bei der Hydrierung von DNT zu TDA ist die weitere Erhöhung der Ausbeute und insbesondere die Verbesserung der Selektivität des Verfahrens, um so die Nebenreaktionen, die zur Bildung von hochmolekularen Nebenprodukten oder zur Bildung von Leichtsiedern führen, zurückzudrängen. Weiterhin sollte der Katalysator auch bei höheren Reaktionstemperaturen stabil sein und keine Verschlechterung der Selektivität des Verfahrens zulassen.

Um eine wirtschaftliche Verfahren zu betrieben, muss das Herstellung und Aufarbeitung des Katalysators möglichst günstig sein. Die Herstellung des Katalysators wird billiger, wenn in möglichst wenigen Herstellungsschritten durchgeführt wird. Die Aufarbeitung von gebrauchten Edelmetallkatalysatoren wird billiger, wenn den Anteil an zusätzlichen Nichtedelmetall Komponenten möglichst niedrig wird.

Aufgabe der Erfindung war es demzufolge, Katalysatoren für die Hydrierung von aromatischen Nitroverbindungen zu den entsprechenden Aminen, insbesondere von DNT zu TDA, bereitzustellen, die zu einer höheren Ausbeute und Selektivität des Verfahrens führen und deren Herstellung sowie Aufarbeitung mit niedrigen Kosten verbunden ist.

Diese Aufgabe konnte überraschenderweise gelöst werden durch die Verwendung von Hydrierkatalysatoren, bestehend aus Platin, Nickel und einem zusätzlichen Element auf einem Träger bei der Hydrierung von aromatischen Nitroverbindungen zu den entsprechenden Aminen.

Gegenstand der Erfindung ist somit Verfahren zur Herstellung von aromatischen Aminen durch katalytische Hydrierung der entsprechenden Nitroverbindungen, insbesondere zur Herstellung von Toluylendiamin durch Hydrierung von Dinitrotoluol, dadurch gekennzeichnet, dass Hydrierkatalysatoren eingesetzt werden, bei denen als aktive Komponente ein Gemisch von Platin, Nickel und einem zusätzlichen Metall auf einem Träger vorliegt.

Gegenstand der Erfindung ist weiterhin die Verwendung von Hydrierkatalysatoren, enthaltend als aktive Komponente ein Gemisch von Platin, Nickel und einem zusätzlichen Metall auf einem Träger, zur Herstellung von aromatischen Aminen durch katalytische Hydrierung der entsprechenden Nitroverbindungen, insbesondere zur Herstellung von Toluylendiamin durch Hydrierung von Dinitrotoluol.

Das zusätzliche Metall ist vorzugsweise ausgewählt aus der Gruppe, enthaltend Kupfer, Kobalt, Eisen, Zink, Mangan und Chrom, insbesondere aus der Gruppe, enthaltend Kupfer, Kobalt, Eisen und Zink.

Die Metallpartikel sind meist polykristallin und können mit einem hoch-auflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂, TiO₂ eingesetzt. Bei Graphit sind die HSAG (High Surface Area Graphite) mit einer Oberfläche von 50 bis 300 m²/g besonders bevorzugt. Besonders bevorzugt sind Aktivkohle, insbesondere die physisch oder chemisch aktivierte Aktivkohle, oder Ruße, wie Acetylenruß.

Die erfindungsgemäß verwendeten Hydrierkatalysatoren enthalten vorzugsweise 1 - 5 Gew.-% Platin, 0,3 bis 1,5 Gew.-% Nickel und 0,05 - 1,5 Gew.-% des Zusatzelements, jeweils bezogen auf das Gewicht des Katalysators. Der Gehalt an Nichtedelmetallen beträgt vorzugsweise maximal 1,6 Gew.-%, insbesondere maximal 0,9 Gew.-%, jeweils bezogen auf das Gewicht des Katalysators.

Bei der Durchführung des erfindungsgemäßen Hydrierverfahrens wird der Katalysator vorzugsweise in einer Menge von 0,01 bis 10, besonders bevorzugt 0,01 bis 5 Gew.-%, insbesondere 0,2 bis 2 Gew.-%, bezogen auf das Reaktionsgemisch, eingesetzt.

Der Katalysator wird zumeist in reduziertem und passiviertem Zustand in den Reaktor eingebracht. Unter dem reduzierten und passivierten Zustand des Katalysators wird verstanden, dass der Katalysator nach der Herstellung aktiviert ist, danach jedoch, aus Sicherheitsgründen, die aktiven Zentren, beispielsweise durch Überleiten von Sauerstoff oder Kohlendioxid, passiviert werden. Alternativ kann der Katalysator unter einer inerten Atmosphäre oder in einem nicht leicht-entzündlichen Lösungsmittel ausgebaut und stabilisiert werden, beispielsweise in Wasser, TDA/Wasser oder höheren Alkoholen, wie Butanol oder Ethylenglykol.

Das erfindungsgemäße Verfahren kann kontinuierlich oder diskontinuierlich unter Verwendung üblicher Reaktoren bei üblichen Verfahrensparametern, wie Druck und Temperatur, durchgeführt werden.

Vorzugsweise wird das Verfahren zur Herstellung von aromatischen Aminen, insbesondere DNT, unter Verwendung der erfindungsgemäßen Katalysatoren bei Drücken im Bereich von 5 bis 100 bar, besonders bevorzugt bei 10 bis 40 bar, insbesondere bei 20 bis 25 bar, durchgeführt.

Vorzugsweise wird das Verfahren zur Herstellung von aromatischen Aminen, insbesondere DNT, unter Verwendung der Katalysatoren bei einer Temperatur im Bereich von 80 bis 250°C, besonders bevorzugt im Bereich von 100 bis 220°C und insbesondere im Bereich 160 bis 200°C durchgeführt.

Zumeist wird die Hydrierung in Form einer kontinuierlichen Suspensionshydrierung in üblichen und geeigneten Reaktoren durchgeführt. Als Reaktoren kommen beispielsweise Rührkessel oder Schlaufenreaktoren, wie zum Beispiel Strahlschlaufenreaktoren, sogenannte Loop-Venturi-Reaktoren, oder Schlaufenreaktoren mit innerer Strömungsumkehr, wie in WO 00/35852 beschrieben, zum Einsatz. Zur Abtrennung der Katalysatoren vom ausgeschleusten Reaktionsgemisch können beispielsweise Querstromfilter eingesetzt werden. Ein derartiges Verfahren ist beispielsweise in WO 03/66571 beschrieben.

Die bei der Hydrierung gebildeten Amine werden dem Hydriervorgang kontinuierlich oder diskontinuierlich entzogen und einer Aufarbeitung, beispielsweise einer destillativen Nachbehandlung, unterworfen.

Vorzugsweise werden im Rahmen des erfindungsgemäßen Verfahrens aromatische Nitroverbindungen mit einer oder mehreren Nitrogruppen und 6 bis 18 C-Atomen, bsp. Nitrobenzole, wie z.B. o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl) nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 5 Prozent an vicinalem DNT und 0,5 bis 1,5 % an 2,5- und 3,5-Dinitrotoluol aufweisen, zu den entsprechenden Aminen hydriert.

Die Katalysatoren könnten bei einem Hydrierverfahren eingesetzt werden, bei dem die aromatische Nitroverbindung in reiner Form, als Mischung mit dem entsprechenden Di- und/oder Polyamin, als Mischung mit dem entsprechenden Di- und/oder Polyamin und Wasser, als Mischung mit dem entsprechenden Di- und/oder Polyamin, Wasser und einem alkoholischen Lösungsmittel oder als Mischung mit dem entsprechenden Di- und/oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Sofern ein oben beschriebenes Gemisch eingesetzt wird, liegt das Verhältnis von Aminverbindung zu Wasser vorzugsweise im Bereich von 10:1 bis 1:10, besonders bevorzugt im Bereich von 4:1 bis 1:1 und das Verhältnis des Amin/Wasser-Gemischs zu mindestens einem alkoholischen Lösungsmittel vorzugsweise bei 1000:1 bis 1:1, besonders bevorzugt bei 50:1 bis 5:1.

Um Nebenreaktionen zu unterdrücken, ist es bevorzugt, das Verfahren so zu führen, dass der Katalysator an seiner Belastungsgrenze gefahren wird. Dies kann beispielsweise durch die Menge der zudosierten Nitroverbindung, die Menge des Katalysators im Reaktionsgemisch, die Temperatur oder den Druck gesteuert werden.

Unter der Belastungsgrenze des Katalysators wird die Menge der hydrierbaren Stickstoff- und Sauerstoffatome enthaltenden Gruppen verstanden, die vom Katalysator bei gegebenen Druck- und Temperaturbedingungen hydriert werden können. Bei den Stickstoff- und Sauerstoffatome enthaltenden Gruppen kann es sich neben Nitrogruppen auch um Nitrosogruppen und Nitrosamingruppen handeln.

Die Herstellung der Katalysatoren erfolgt beispielsweise, indem der Träger vorgelegt und mit einer wässrigen Lösung der Platin- und Nickelsalze zusammen mit dem Zusatzelement gebracht wird. Dabei sollte die Menge des zur Lösung der Salze verwendeten Wassers so bemessen sein, dass eine knetbare Paste entsteht. Vorzugsweise wird das Wasser in einer Menge von 100 bis 200 Gew.-% der Trägermasse eingesetzt. Als Metallsalze kommen insbesondere Nitrate oder Chloride zum Einsatz, wobei Nitrate wegen ihrer geringeren Korrosivität bevorzugt sind. Die Paste wird gemischt und danach das Wasser bei geringem Druck und Temperaturen im Bereich zwischen 50 und 100°C verdampft, beispielsweise in einem Rotationsverdampfer oder einem Ofen. Aus Sicherheitsgründen kann das Verdampfen in einem Stickstoffstrom erfolgen. Die Fixierung der Metalle auf dem Träger bei der Verwendung von Chloriden als Metallsalze durch Reduktion mit Wasserstoff erfolgen. Hierbei kann jedoch Korrosion auftreten. Bevorzugt werden die Metalle daher alkalisch fixiert. Dies erfolgt insbesondere durch Zugabe einer wässrigen Lösung von Alkalicarbonaten und nachfolgendes anionfrei waschen des Trägers. Alternativ können die Metalle auch alkalisch, insbesondere bei einem pH-Wert im Bereich von 8 bis 9, aus einer überstehen-den Lösung auf den Träger gefällt werden. Danach wird der Träger getrocknet, vorzugsweise wie oben beschrieben, und mit Wasserstoff reduziert. Dies kann beispielsweise in einem Drehkugelofen. Vor dem Ausbau des Katalysators wird dieser passiviert, beispielsweise unter einem Inertgas, wie Stickstoff, der Spuren von Luft, vorzugs-weise nicht mehr als 10 Vol.-%, enthält.

Die nach diesem Verfahren hergestellten Hydrierkatalysatoren enthalten vorzugsweise 1-5 Gew.-% Platin, 0,3-1,5 Gew.-% Nickel und 0,2-1,0 Gew.-% des Zusatzelementes. Insbesondere enthalten sie höchstens 0,9 Gew.-% Nichtedelmetalle.

In einer anderen Ausführungsform der Herstellung der Hydrierkatalysatoren erfolgt die Reduktion der Katalysatoren durch Zusatz von reduzierend wirkenden Salzen, wie Ammoniumcarboxylaten, beispielsweise Ammoniumformiat. Dazu wird der Träger mit Wasser suspendiert und gleichzeitig oder nach der Suspendierung die Lösungen der Metallsalze zugegeben. Zu dieser Lösung werden die reduzierend wirkenden Salze zugegeben und die Suspension erhitzt, beispielsweise durch Kochen unter Rückfluss. Anschließend werden die Katalysatoren wie oben beschrieben gewaschen und als feuchte Paste verwendet.

Die nach diesem Verfahren hergestellten Hydrierkatalysatoren enthalten vorzugsweise 1-5 Gew.-% Platin, 0,3-1,5 Gew.-% Nickel und 0,05-0,5 Gew.-% des Zusatzelementes. Insbesondere enthalten sie höchstens 0,9 Gew.-% Nichtedelmetalle.

Durch die Verwendung der Katalysatoren ist es möglich, die Hydrierung von DNT zu TDA bei Temperaturen im Bereich zwischen 120 und 250°C, insbesondere 120 bis 150°C, bei denen sich bei Verwendung herkömmlicher Katalysatoren die Selektivität der Umsetzung stark verschlechtert, durchzuführen. Eine Erhöhung der Reaktionstemperatur ist vorteilhaft da die Löslichkeiten der einzelnen Komponente höher ist, als auch nimmt die Reaktionsgeschwindigkeit mit der Temperatur zu. Man kann also die RZA (Raum Zeit Ausbeute) erhöhen, solange die Reaktionsenergie sicher abgeführt werden kann.

Die Erfindung soll in den folgenden Beispielen näher erläutert werden.

### Beispiel 1

Ein Aktivkohleträger Norit® SX + wurde in einer Schale vorgelegt und Pt(II)Nitrat für 3 Gew.-% Platin, bezogen auf das Gewicht des Katalysators, Ni(II)Nitrat-Hexahydrat für 0,9 Gew.-% Nickel und Cu(II)Nitrat-Hemipentahydrat für 0,2 Gew.-% Kupfer, bezogen auf das Gewicht des Katalysators, wurden in Wasser in einer Menge von 200 Gew.-% der Menge des Trägers gelöst und zu dem Träger gegeben, so dass eine knetbare Paste entstand. Die Paste wurde gut gemischt. Das Lösungsmittel Wasser wurde in einem Rotationsverdampfer unter leichtem Sieden bei 60-70°C und einem Druck von 0,2 bis 0,4 bar verdampft. Die Metalle wurden auf den Träger durch Zugabe einer Lösung von Natriumcarbonat in einer Menge von 16 Gew.-% der Trägermenge in 200 Gew.-% der Trägermenge Wasser, alkalisch fixiert, und die Probe nitratfrei gewaschen. Der so erhaltene Katalysator wurde bei 80° C getrocknet, bevor er 4 Stunden bei 400° C unter Wasserstoffstrom in einem Drehkugelofen reduziert wurde. Vor dem Ausbau wurde der Katalysator in verdünnter Luft (5 Vol.-% Luft in Stickstoff) bei Raumtemperatur passiviert. Der so erhaltene Katalysator wird als Katalysator 1 bezeichnet. Der so erhaltene Katalysator hatte einen Gehalt von 2,5 Gew.-% Platin, 0,75 Gew.-% Nickel und 0,13 Gew.-% Kupfer.

### Beispiel 2

Es wurde verfahren wie in Beispiel 1, es wurde jedoch als Zusatzelement 0,2 Gew. % Kobalt in Form des Kobalt(II)-Nitrat Hexahydrat zugesetzt. Der so erhaltene Katalysator wird als Katalysator 2 bezeichnet. Der so erhaltene Katalysator hatte einen Gehalt von 2,9 Gew.-% Platin, 0,8 Gew.-% Nickel und 0,1 Gew.-% Kobalt.

### Beispiel 3

Es wurde verfahren wie in Beispiel 1, es wurde jedoch 0,4 Gew.-% Nickel und als Zusatzelement 0,25 Gew. % Zink in Form des Zink(II)-Nitrat Hexahydrat zugesetzt. Der so erhaltene Katalysator wird als Katalysator 3 bezeichnet. Der so erhaltene Katalysator hatte einen Gehalt von 2,7 Gew.-% Platin, 0,39 Gew.-% Nickel und 0,25 Gew.-% Zink.

### Beispiel 4

Der in Beispiel 1 verwendete Träger wurde in Wasser zu einer 10 %igen Suspension suspendiert. Dazu wurden die in Beispiel 1 beschriebenen Metallsalze in einem Verhältnis 0,7 Gew.% Nickel und 0,2 Gew.% Kupfer gegeben und mit Ammoniumformiat unter Rückfluss für 2 Stunden gekocht. Der so erhaltene Katalysator wurde nitratfrei gewaschen. Der so erhaltene Katalysator wird als Katalysator 4 bezeichnet. Der so erhaltene Katalysator hatte einen Gehalt von 2,9 Gew.-% Platin, 0,65 Gew.-% Nickel und 0,22 Gew.-% Kupfer.

### Beispiel 5

Es wurde verfahren wie in Beispiel 4, es wurde jedoch 0,45 Gew.-% Nickel und als Zusatzelement 0,2 Gew. % Kobalt zugesetzt. Der so erhaltene Katalysator wird als Katalysator 5 bezeichnet. Der so erhaltene Katalysator hatte einen Gehalt von 3,0 Gew.-% Platin, 0,38 Gew.-% Nickel und 0,09 Gew.-% Kobalt.

### Beispiel 6

Es wurde verfahren wie in Beispiel 4, es wurde jedoch 0,45 Gew.-% Nickel und als Zusatzelement 0,2 Gew. % Eisen zugesetzt. Der so erhaltene Katalysator wird als Katalysator 6 bezeichnet. Der so erhaltene Katalysator hatte einen Gehalt von 3,0 Gew.-% Platin, 0,40 Gew.-% Nickel und 0,15 Gew.-% Eisen.

### Beispiel 7 (Vergleich 1)

Kommerzieller Nickelkatalysator auf einem ZrO₂-Träger.

### Beispiel 8 (Vergleich 2) (5 % Pd/C)

Kommerzieller, 5 Gew.-% auf Aktivkohle enthaltender Pd Referenzkatalysator (50 % wasserfeucht).

### Beispiel 9 (Vergleich 3)

Es wurde verfahren wie in Beispiel 4, es wurde jedoch kein Nickelsalz zugesetzt. Der so erhaltene Katalysator wird als Katalysator 9 bezeichnet.

### Beispiel 10 (Vergleich 4)

Es wurde verfahren wie in Beispiel 1, es wurde jedoch nur 1,0 Gew.-% Nickel zugesetzt. Der so erhaltene Katalysator wird als Katalysator 10 bezeichnet. Der Metallgehalt betrug 2,9 Gew.-% Platin und 0,97 Gew.-% Nickel.

### Beispiel 11 (Vergleich 5)

Es wurde verfahren wie in Beispiel 4, es wurde jedoch 1,0 Gew.-% Palladium in Form des Palladium(II)-Nitrat, 15 Gew.-% Nickel und 1,0 Gew.-% Zink zugesetzt. Der so erhaltene Katalysator wird als Katalysator 11 bezeichnet. Der Metallgehalt betrug 0,92 Gew.-% Palladium, 13,8 Gew.-% Nickel und 0,96 Gew.-% Zink.

### Hydrierung von DNT zu TDA

Die Hydrierung von DNT zu TDA, wurde in einem 300 ml kontinuierlichen Rührkessel durchgeführt, der Katalysator wurde mechanisch im Reaktor zurückgehalten. Der Katalysator wurde im Wasser suspendiert und in den Reaktor gebracht (Katalysatormenge 1 bis 2 Gew.-% des Flüssigkeitsvolumens des Reaktors), auf eine Temperatur von 125°C gebracht. Unter einem Wasserstoff-Druck von 22 bar wurde DNT in einer solchen Menge kontinuierlich als Schmelze zudosiert, dass eine Raum-Zeit-Ausbeute von 150-600 Kg_{TDA}/m³.h eingestellt wurde. Proben wurden mittels Gaschromatographie analysiert: Die TDA Ausbeute, Bildung von Hochsiedern und Leichtsiedern wurde verfolgt.

Die Katalysatoren, deren Zusammensetzung sowie die Ergebnisse sind der Tabelle 1 zu entnehmen.

| Beispiel | Katalysator | Katalysator - Herkunft oder Herstellungsmethode | Edelmetall % | Nichtedelmetall % | RZA (kg_{TDA}/m³h) | TDA Sel % |
|---|---|---|---|---|---|---|
| 1 | 2,5%Pt-0,75%Ni-0,13%Cu | 2-stufig, Bsp. 1 | 2,5 | 0,88 | 150-400 | 99,62 |
| 2 | 2,9%Pt-0,80%Ni-0,10%Co | 2-stufig, Bsp. 1 | 2,9 | 0,90 | 200-600 | 99,54 |
| 3 | 2,7%Pt-0,39%Ni-0,25%Zn | 2-stufig, Bsp. 1 | 2,7 | 0,64 | 200-400 | 99,60 |
| 4 | 2,9%Pt-0,65%Ni-0,22%Cu | 1-stufig, Bsp. 4 | 2,9 | 0,87 | 200-600 | 99,56 |
| 5 | 3,0%Pt-0,38%Ni-0,09%Co | 1-stufig, Bsp. 4 | 3,0 | 0,47 | 400-600 | 99,53 |
| 6 | 3,0%Pt-0,40%Ni-0,15%Fe | 1-stufig, Bsp. 4 | 3,0 | 0,55 | 400-600 | 99,53 |
| 7 | 65%Ni/ZrO2 | Komerziell | 0,0 | 65,00 | 150-600 | 99,30 |
| 8 | 5%Pd | Komerziell | 5,0 | 0 | 150-600 | ca. 95 |
| 9 | 3% Pt | 1-stufig, Bsp. 4 | 3,0 | 0 | 150-600 | 99,00 |
| 10 | 2,9%Pt-0,97%Ni | 2-stufig, Bsp. 1 | 2,9 | 0,97 | 400-600 | 99,50 |
| 11 | 0,92%Pd-13,8%Ni-0,96%Zn | 1-stufig, Bsp. 4 | 0,9 | 15,68 | 150-600 | 99,21 |

Die Beispiele zeigen, dass die monometallische Katalysatoren (7-9) Ausbeuten ergeben, die denen der erfindungsgemäßen Katalysatoren (1-6) deutlich unterlegen sind. Mit den trimetallischen Katalysatoren 1-3, die zweistufig laut Beispiel 1 hergestellt werden, kann eine hohe TDA Selektivität erreicht werden, die dem bimetallischen Pt-Ni Katalysator 10 sowie auch dem trimetallischem Pd-Ni-Zn Katalysator 11 überlegen ist. Mit den trimetallischen Katalysatoren 4-6, die einstufig hergestellt wurden, konnten TDA-Selektivitäten erreicht werden, die mit den aus dem Stand der Technik bekannten bimetallischem Pt-Ni Katalysatoren (10) vergleichbar sind. Der Gehalt an Nichtedelmetallen im Katalysatoren 3, 5-6 ist jedoch niedriger als in dem Vergleichskatalysator 10, daher sollte die Aufarbeitung von diesem Katalysator nach seinem Gebrauch günstiger sein.

## Patentansprüche

1. Verfahren zur Herstellung von aromatischen Aminen durch katalytische Hydrierung der entsprechenden Nitroverbindungen, insbesondere zur Herstellung von Toluylendiamin durch Hydrierung von Dinitrotoluol, **dadurch gekennzeichnet, dass** Hydrierkatalysatoren eingesetzt werden, bei denen als aktive Komponente ein Gemisch von Platin, Nickel und einem zusätzlichen Metall auf einem Träger vorliegt.

2. Verfahren nach Anspruch 1, **dadurch gekennzeichnet, dass** das zusätzliche Metall ausgewählt ist aus der Gruppe, enthaltend Kupfer, Kobalt, Eisen, Zink, Mangan und Chrom.

3. Verfahren nach Anspruch 1, **dadurch gekennzeichnet, dass** das zusätzliche Metall ausgewählt ist aus der Gruppe, enthaltend Kupfer, Kobalt, Eisen und Zink.

4. Verfahren nach Anspruch 1, **dadurch gekennzeichnet, dass** der Träger ausgewählt ist aus der Gruppe enthaltend Aktivkohle, Ruß, Graphit und Metalloxide.

5. Verfahren nach Anspruch 1, **dadurch gekennzeichnet, dass** der Katalysator 1 - 5 Gew.-% Platin, 0,3 bis 1,5 Gew.-% Nickel und 0,05 - 1,5 Gew.-% des Zusatzelements, jeweils bezogen auf das Gewicht des Katalysators, enthält.

6. Verfahren nach Anspruch 1, **dadurch gekennzeichnet, dass** der Gehalt an Nichtedelmetallen auf dem Katalysator maximal 0,9% beträgt.

7. Verfahren nach Anspruch 1, **dadurch gekennzeichnet, dass** der Katalysator in einer Menge von 0,01 bis 5 Gew.-%, bezogen auf die Reaktionsmischung, eingesetzt wird.

8. Verwendung von Katalysatoren, bei denen als aktive Komponente ein Gemisch von Platin, Nickel und einem zusätzlichen Metall auf einem Träger vorliegt, zur Hydrierung von Dinitrotoluol.

## Claims

1. A process for preparing aromatic amines by catalytically hydrogenating the corresponding nitro compounds, especially for preparing tolylenediamine by hydrogenating dinitrotoluene, which comprises using hydrogenation catalysts in which the active component present is a mixture of platinum, nickel and an additional metal on a support.

2. The process according to claim 1, wherein the additional metal is selected from the group comprising copper, cobalt, iron, zinc, manganese and chromium.

3. The process according to claim 1, wherein the additional metal is selected from the group comprising copper, cobalt, iron and zinc.

4. The process according to claim 1, wherein the support is selected from the group comprising activated carbon, carbon black, graphite and metal oxides.

5. The process according to claim 1, wherein the catalyst comprises 1-5% by weight of platinum, from 0.3 to 1.5% by weight of nickel and 0.05-1.5% by weight of the additional element, based in each case on the weight of the catalyst.

6. The process according to claim 1, wherein the content of base metals in the catalyst is not more than 0.9%.

7. The process according to claim 1, wherein the catalyst is used in an amount of from 0.01 to 5% by weight based on the reaction mixture.

8. The use of catalysts in which the active component present is a mixture of platinum, nickel and an additional metal on a support for hydrogenating dinitrotoluene.

## Revendications

1. Procédé de fabrication d'amines aromatiques par hydrogénation catalytique des composés nitro correspondants, notamment pour la fabrication de toluylène-diamine par hydrogénation de dinitrotoluène, **caractérisé en ce que** des catalyseurs d'hydrogénation dans lesquels un mélange de platine, de nickel et d'un métal supplémentaire est présent sur un support en tant que composant actif sont utilisés.

2. Procédé selon la revendication 1, **caractérisé en ce que** le métal supplémentaire est choisi dans le groupe contenant le cuivre, le cobalt, le fer, le zinc, le manganèse et le chrome.

3. Procédé selon la revendication 1, **caractérisé en ce que** le métal supplémentaire est choisi dans le groupe contenant le cuivre, le cobalt, le fer et le zinc.

4. Procédé selon la revendication 1, **caractérisé en ce que** le support est choisi dans le groupe contenant le charbon actif, le noir de carbone, le graphite et les oxydes métalliques.

5. Procédé selon la revendication 1, **caractérisé en ce que** le catalyseur contient 1 à 5 % en poids de platine, 0,3 à 1,5 % en poids de nickel et 0,05 à 1,5 % en poids de l'élément supplémentaire, à chaque fois par rapport au poids du catalyseur.

6. Procédé selon la revendication 1, **caractérisé en ce que** la teneur en métaux non nobles sur le catalyseur est d'au plus 0,9 %.

7. Procédé selon la revendication 1, **caractérisé en ce que** le catalyseur est utilisé en une quantité de 0,01 à 5 % en poids, par rapport au mélange réactionnel.

8. Utilisation de catalyseurs dans lesquels un mélange de platine, de nickel et d'un métal supplémentaire est présent sur un support en tant que composant actif, pour l'hydrogénation de dinitrotoluène.
